# EUROPEAN PATENT SPECIFICATION

(11) **EP 1 905 285 B1**
(45) Date of publication and mention of the grant of the patent: **14.10.2015**
(21) Application number: 06762496.5
(22) Date of filing: 07.07.2006
(51) Int. Cl.: H05H 1/34, H05H 1/28

(54) **PLASMA SURGICAL DEVICE**
CHIRURGISCHE PLASMAEINRICHTUNG
DISPOSITIF CHIRURGICAL AU PLASMA

(30) Priority: 08.07.2005 SE 0501603
(43) Date of publication of application: 02.04.2008
(73) Proprietor: Plasma Surgical AB, 431 35 Mölndal (SE); Plasma Surgical Investments Limited, Tortola (VG)
(72) Inventor: SUSLOV, Nickolay, 421 67 Västra Frölunda (SE); RUBINER, Igor, 427 37 Billdal (SE)
(74) Representative: Somlo, Tommy
(86) International application number: PCT/EP2006/006689
(87) International publication number: WO 2007/006517

(56) References cited:
- EP-A1- 0 748 149
- WO-A-02/30308
- WO-A-2004/105450
- WO-A1-2004/030551
- US-A- 4 743 734
- US-A- 5 620 616

## Description

### CLAIM OF PRIORITY

This application claims priority of a Swedish Patent Application No. 0501603-5 filed on July 8, 2005.

### FIELD OF THE INVENTION

The present invention relates to a plasma surgical device comprising a plasma-generating device, the plasma-generating device comprising an anode, a cathode and at least one intermediate electrode, said intermediate electrode being arranged at least partly between said anode and said cathode, and said intermediate electrode and said anode forming at least a part of a plasma channel which has an opening in said anode.

### BACKGROUND ART

Plasma devices relate to the devices which are arranged to generate a gas plasma. Plasma devices, such as WO 2004/105450 (Bijker et al.) are known for use in industrial applications. WO 2004/105450 discloses a cascade source provided with a cathode housing, a number of insulated cascade plates which together bound a plasma channel, and an anode plate provided with an outflow opening connected to the plasma channel. In other plasma devices such gas plasma can be used, for instance, in surgery for the purpose of causing destruction (dissection) and/or coagulation of biological tissues.

As a rule, such plasma devices are formed with a long and narrow end or the like which can easily be applied to a desired area that is to be treated, such as bleeding tissue. At the tip of the device, a gas plasma is present, the high temperature of which allows treatment of the tissue adjacent to the tip.

WO 2004/030551 (Suslov) discloses a plasma surgical device according to prior art. This device comprises a plasma-generating system with an anode, a cathode and a gas supply channel for supplying gas to the plasma-generating system. Moreover the plasma-generating system comprises a plurality of electrodes which are arranged between said cathode and anode. A housing of an electrically conductive material which is connected to the anode encloses the plasma-generating system and forms the gas supply channel.

Owing to the recent developments in surgical technology, that referred to as laparoscopic (keyhole) surgery is being used more often. This implies, for example, a greater need for devices with small dimensions to allow accessibility without extensive surgery. Small instruments are also advantageous in surgical operations to achieve good accuracy.

It is also desirable to be able to improve the accuracy of the plasma jet in such a manner that, for example, smaller areas can be affected by heat. It is also desirable to be able to obtain a plasma-generating device which gives limited action of heat around the area which is to be treated.

Thus, there is a need for improved plasma devices, in particular plasma devices with small dimensions and great accuracy which can produce a high temperature plasma.

### SUMMARY OF THE INVENTION

An object of the present invention is to provide a plasma surgical device.

According to the invention, a plasma surgical device is provided as defined in claim 1.

According to the invention, the plasma-generating device comprises at least one coolant channel which is arranged with at least one outlet opening which is positioned beyond, in the direction from the cathode to the anode, said at least one intermediate electrode, and the channel direction of said coolant channel at said outlet opening has a directional component which is the same as that of the channel direction of the plasma channel at the opening thereof.

This construction of the plasma-generating device allows that a coolant, which is adapted to flow in the coolant channel, is allowed to flow out at the end of the plasma-generating device in the vicinity of the opening of the plasma channel. An advantage achieved by this arrangement is that a coolant flowing out through an outlet of the coolant channel can be used to screen and restrict a plasma jet which is emitted through the plasma channel outlet which opens into the anode. Screening and restriction of the plasma jet allows, inter alia, advantages in treatment of above all small areas since the active propagations of the plasma-generating jet can be limited.

It is also possible to use the coolant flowing out to cool an object affected by the plasma jet. Cooling of the object that is to be treated can, for instance, be suitable to protect regions surrounding the area of treatment.

For instance, the plasma jet can be screened in its longitudinal direction so that there is substantially low heat on one side of the screen and substantially high heat on the other side of the screen. In this manner, a substantially distinct position of the plasma jet is obtained, in the flow direction of the plasma jet, where the object to be treated is affected, which can provide improved accuracy in operation of the plasma-generating device.

Similarly, the coolant flowing out can provide screening of the plasma jet in the radial direction relative to the flow direction of the plasma jet. Screening in the radial direction in this way allows that a relatively small surface can be affected by heat in treatment. Screening in the lateral direction, relative to the flow direction of the plasma, can also allow that areas around the treated region can at the same time be cooled by the coolant flowing out and thus be affected to a relatively small extent by the heat of the plasma jet.

Prior art plasma-generating devices usually have a closed coolant system for cooling the plasma-generating device in operation. Such a closed coolant system is often arranged by the coolant flowing in along one path in the plasma-generating device and returning along another path. This often causes relatively long flow paths. A drawback of long flow paths is that flow channels for the coolant must frequently be made relatively large to prevent extensive pressure drops. This means in turn that the flow channels occupy space that affects the outer dimensions of the plasma-generating device.

A further advantage of the invention is that pressure drops in the coolant channel can be reduced compared with, for instance, closed and circulating coolant systems. Consequently the cross-section of the coolant channel can be kept relatively small, which means that also the outer dimensions of the plasma-generating device can be reduced. Reduced dimensions of the plasma-generating device are often desirable in connection with, for instance, use in space-limited regions or in operation that requires great accuracy. Suitably the end of the plasma-generating device next to the anode ("the anode end of the device") has an outer dimension which is less than 10 mm, preferably less than 5 mm. In an alternative embodiment, the outer dimension of the plasma-generating device is equal to or less than 3 mm. The anode end of the device preferably has a circular outer geometry.

Thus, the invention allows that the coolant which is adapted to flow through the coolant channel can be used to cool the plasma-generating device in operation, screen and limit the propagation of the plasma jet and cool regions surrounding the area affected by the plasma jet. However, it will be appreciated that, dependent on the application, it is possible to use individual fields of application or a plurality of these fields of application.

To allow the coolant in the coolant channel to flow out in the vicinity of the plasma jet, it is advantageous to arrange the outlet opening of the coolant channel beside and spaced from the opening of the plasma channel.

In one embodiment, the opening of the coolant channel is arranged in the anode.

By arranging the outlet opening of the coolant channel and the opening of the plasma channel close to each other, the end of the plasma-generating device has in the vicinity of the anode a nozzle with at least two outlets for discharging coolant and plasma, respectively. It is suitable to let the coolant channel extend along the whole anode, or parts of the anode, to allow also cooling of the anode in operation. In one embodiment, the outlet of the coolant channel is arranged on the same level as, or in front of, in the direction from the cathode to the anode, the outlet of the plasma channel in the anode.

The main extent of the coolant channel is suitably substantially parallel to said plasma channel. By arranging the coolant channel parallel to the plasma channel, it is possible to provide, for instance, a compact and narrow plasma-generating device. The coolant channel suitably consists of a throughflow channel whose main extent is arranged in the longitudinal direction of the plasma channel. With such a design, the coolant can, for instance, be supplied at one end of the plasma-generating device so as to flow out at the opposite end next to the anode.

Depending on desirable properties of the plasma-generating device, an outlet portion of the coolant channel can be directed and angled in different suitable ways. In one embodiment of the plasma-generating device, the channel direction of the coolant channel at the outlet opening can extend, in the direction from the cathode to the anode, at an angle between +30 and -30 degrees in relation to the channel direction of said plasma channel at the opening thereof. By choosing different angles for different plasma-generating devices, the plasma jet can thus be screened and restricted in various ways both in its longitudinal direction and transversely to its longitudinal direction. The above stated suitable variations of the channel direction of the coolant channel in relation to the channel direction of the plasma channel are such that an angle of 0 degrees corresponds to the fact that the channel directions of both channels are parallel.

In the case that a restriction is desired in the lateral direction, radially transversely to the longitudinal direction of the plasma channel, of the plasma jet, the channel direction of the coolant channel at said outlet opening can extend, in the direction from the cathode to the anode, substantially parallel to the channel direction of said plasma channel at the opening thereof.

In another embodiment, a smaller radial restriction transversely to the longitudinal direction of the plasma channel can be desirable. For an alternative embodiment, for instance, the channel direction of the coolant channel at said outlet opening can extend, in the direction from the cathode to the anode, at an angle away from the channel direction of said plasma channel at the opening thereof.

In another alternative embodiment, the channel direction of the coolant channel at said outlet opening can extend, in the direction from the cathode to the anode, at an angle towards the channel direction of said plasma channel at the opening thereof. This embodiment allows, for instance, that the plasma jet can be restricted, by the coolant flowing out, both in the lateral direction of the flow direction of the plasma jet and in the longitudinal direction of the flow direction of the plasma jet.

It will be appreciated that an outlet portion of the coolant channel can be arranged in various ways depending on the properties and performance that are desired in the plasma-generating device. It will also be appreciated that the plasma-generating device can be provided with a plurality of such outlet portions. A plurality of such outlet portions can be directed and angled in a similar manner. However, it is also possible to arrange a plurality of different outlet portions with different directions and angles relative to the channel direction of the plasma channel at the opening thereof.

The plasma-generating device can also be provided with one or more coolant channels. Moreover each such coolant channel can be provided with one or more outlet portions.

In use, the coolant channel is preferably passed by a coolant which flows from the cathode to the anode. As coolant, use is preferably made of water, although other types of fluids are possible. Use of a suitable coolant allows that heat emitted from the plasma-generating device in operation can be absorbed and extracted.

To provide efficient cooling of the plasma-generating device, it may be advantageous that a part of said coolant channel extends along said at least one intermediate electrode. By the coolant in the coolant channel being allowed to flow in direct contact with the intermediate electrode, good heat transfer between the intermediate electrode and the coolant is thus achieved. For suitable cooling of large parts of the intermediate electrode, a part of said coolant channel can extend along the outer periphery of said at least one intermediate electrode. For example, the coolant channel surrounds the outer periphery of said at least one intermediate electrode.

In one embodiment, an end sleeve of the plasma-generating device, which end sleeve preferably is connected to the anode, constitutes part of a radially outwardly positioned boundary surface of the coolant channel. In another alternative embodiment, said at least one intermediate electrode constitutes part of a radially inwardly positioned boundary surface of the coolant channel. By using these parts of the structure of the plasma-generating device as a part of the boundary surfaces of the coolant channel, good heat transfer can be obtained between the coolant and adjoining parts that are heated in operation. Moreover the dimensions of the plasma-generating device can be reduced by the use of separate coolant channel portions being reduced.

It is advantageous to arrange the coolant channel so that, in use, it is passed by a coolant quantity of between 1 and 5 ml/s. Such flow rates are especially advantageous in surgical applications where higher flow rates can be detrimental to the patient.

To allow the coolant to be distributed around the plasma jet, it may be advantageous that at least one coolant channel is provided with at least two outlets, preferably at least four outlets. Moreover the plasma-generating device can suitably be provided with a plurality of coolant channels. The number of coolant channels and the number of outlets can be optionally varied, depending on the field of application and the desired properties of the plasma-generating device.

According to the invention, a plasma surgical device is provided, comprising a plasma-generating device as described above. Such a plasma surgical device of the type here described can suitably be used for destruction or coagulation of biological tissue. Moreover, such a plasma surgical device can advantageously be used in heart or brain surgery. Alternatively such a plasma surgical device can advantageously be used in liver, spleen, kidney surgery or in skin treatment in plastic and cosmetic surgery.

### BRIEF DESCRIPTION OF THE DRAWINGS

The invention will now be described in more detail with reference to the accompanying schematic drawings which by way of example illustrate currently preferred embodiments of the invention.
Fig. 1a is a cross-sectional view of an embodiment of a plasma-generating device according to the invention;
Fig. 1b is a partial enlargement of the embodiment according to Fig. 1a;
Fig. 2a is a cross-sectional view of an alternative embodiment of the plasma-generating device;
Fig. 2b is a front plan view of the plasma-generating device according to Fig. 2a;
Fig. 2c is a front plan view of an alternative embodiment of the plasma-generating device according to Fig. 2a; and
Fig. 3 is a cross-sectional view of another alternative embodiment of a plasma-generating device.

### DESCRIPTION OF PREFERRED EMBODIMENTS

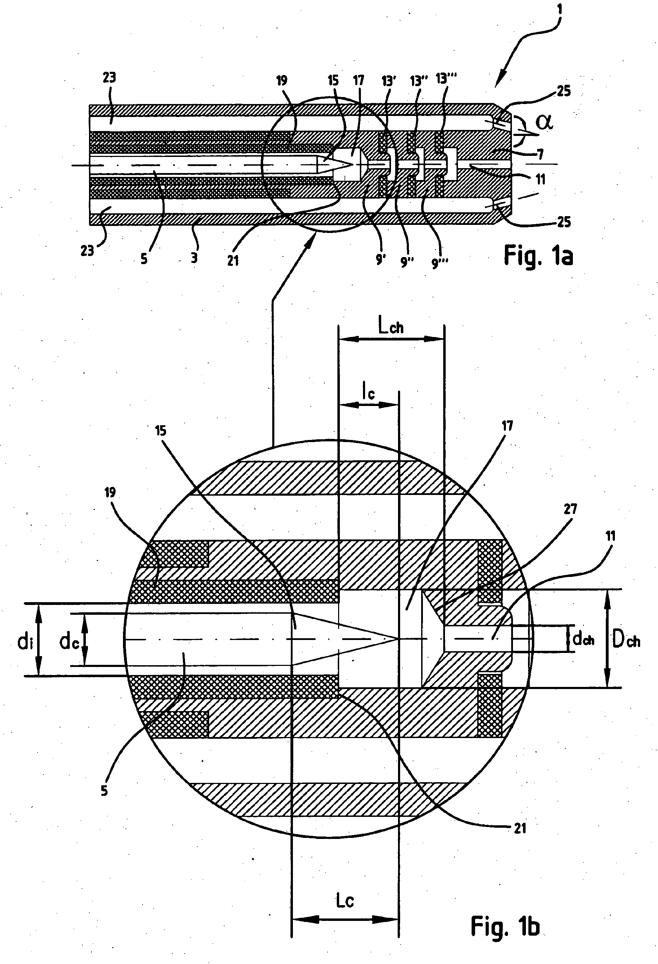

Fig. 1a shows in cross-section an embodiment of a plasma-generating device 1 according to the invention. The cross-section in Fig. 1 a is taken through the centre of the plasma-generating device 1 in its longitudinal direction. The device comprises an elongate end sleeve 3 which accommodates a plasma-generating system for generating plasma which is discharged at the end of the end sleeve 3. The generated plasma can be used, for instance, to stop bleedings in tissues, vaporise tissues, cut tissues etc.

The plasma-generating device 1 according to Fig. 1a comprises a cathode 5, an anode 7 and a number of electrodes 9', 9", 9'" arranged between the anode and the cathode, in this text referred to as intermediate electrodes. The intermediate electrodes 9', 9", 9"' are annular and form part of a plasma channel 11 which extends from a position in front of the cathode 5 and further towards and through the anode 7. The inlet end of the plasma channel 11 is the end closest to the cathode 5; the plasma channel extends through the anode 7 where its outlet opening is arranged. A plasma is intended to be heated in the plasma channel 11 so as to finally flow out through the opening of the plasma channel in the anode 7. The intermediate electrodes 9', 9", 9'" are insulated and spaced from each other by an annular insulator means 13', 13", 13"'. The shape of the intermediate electrodes 9', 9", 9'" and the dimensions of the plasma channel 11 can be adjusted to any desired purposes. The number of intermediate electrodes 9', 9", 9'" can also be optionally varied. The embodiment shown in Fig. 1a is provided with three intermediate electrodes 9', 9", 9"'.

In the embodiment shown in Fig. 1a, the cathode 5 is formed as an elongate cylindrical element. Preferably the cathode 5 is made of tungsten with optional additives, such as lanthanum. Such additives can be used, for instance, to lower the temperature occurring at the end of the cathode 5.

Moreover the end 15 of the cathode 5 which is directed to the anode 7 has a tapering end portion. This tapering portion 15 suitably forms a tip positioned at the end of the cathode as shown in Fig. 1a. The cathode tip 15 is suitably conical in shape. The cathode tip 15 can also consist of a part of a cone or have alternative shapes with a tapering geometry towards the anode 7.

The other end of the cathode 5 which is directed away from the anode 7 is connected to an electrical conductor to be connected to an electric energy source. The conductor is suitably surrounded by an insulator. (The conductor is not shown in Fig. 1a.)

Connected to the inlet end of the plasma channel 11, a plasma chamber 17 is arranged, which has a cross-sectional surface, transversely to the longitudinal direction of the plasma channel 11, which exceeds the cross-sectional surface of the plasma channel 11 at the inlet end thereof. The plasma chamber 17 which is shown in Fig. 1a is circular in cross-section, transversely to the longitudinal direction of the plasma channel 11, and has an extent L_{ch} in the longitudinal direction of the plasma channel 11 which corresponds approximately to the diameter D_{ch} of the plasma chamber 17. The plasma chamber 17 and the plasma channel 11 are substantially concentrically arranged relative to each other. The cathode 5 extends into the plasma chamber 17 at least half the length L_{ch} thereof and the cathode 5 is arranged substantially concentrically with the plasma chamber 17. The plasma chamber 17 consists of a recess formed by the first intermediate electrode 9' which is positioned next to the cathode 5.

Fig. 1 a also shows an insulator element 19 which extends along and around parts of the cathode 5. The insulator element 19 is suitably formed as an elongate cylindrical sleeve and the cathode 5 is partly positioned in a circular hole extending through the tubular insulator element 19. The cathode 5 is substantially centred in the through hole of the insulator element 19. Moreover the inner diameter of the insulator element 19 slightly exceeds the outer diameter of the cathode 5, thereby forming a distance between the outer circumferential surface of the cathode 5 and the inner surface of the circular hole of the insulator element 19.

Preferably the insulator element 19 is made of a temperature-resistant material, such as ceramic material, temperature-resistant plastic material or the like. The insulator element 19 intends to protect adjoining parts of the plasma-generating device from high temperatures which can occur, for instance, around the cathode 5, in particular around the tip 15 of the cathode.

The insulator element 19 and the cathode 5 are arranged relative to each other so that the end 15 of the cathode 5 which is directed to the anode projects beyond an end face 21, which is directed to the anode 7, of the insulator element 19. In the embodiment shown in Fig. 1a, approximately half the tapering tip 15 of the cathode 5 projects beyond the end face 21 of the insulator element 19.

A gas supply part (not shown in Fig. 1a) is connected to the plasma-generating part. The gas supplied to the plasma-generating device 1 advantageously consists of the same type of gases that are used as plasma-generating gas in prior art instruments, for instance inert gases, such as argon, neon, xenon, helium etc. The plasma-generating gas is allowed to flow through the gas supply part and into the space arranged between the cathode 5 and the insulator element 19. Consequently the plasma-generating gas flows along the cathode 5 inside the insulator element 19 towards the anode 7. As the plasma-generating gas passes the end 21 of the insulator element 19, the gas is passed on to the plasma chamber 17.

The plasma-generating device 1 further comprises one or more coolant channels 23 which open into the elongate end sleeve 3. The coolant channels 23 are suitably partly made in one piece with a housing (not shown) which is connected to the end sleeve 3. The end sleeve 3 and the housing can, for instance, be interconnected by a threaded joint, but also other connecting methods, such as welding, soldering etc, are conceivable. Moreover the end sleeve suitably has an outer dimension which is less than 10 mm, preferably less than 5 mm, in particular between 3 mm and 5 mm. At least a housing portion positioned next to the end sleeve suitably has an outer shape and dimension which substantially corresponds to the outer dimension of the end sleeve. In the embodiment of the plasma-generating device shown in Fig. 1a, the end sleeve is circular in cross-section transversely to its longitudinal direction.

The coolant channels 23 suitably consist of through-flow channels which extend through the device and open into or in the vicinity of the anode 7. Moreover parts of such coolant channels 23 can be made, for instance, by extrusion of the housing or mechanical working of the housing. However, it will be appreciated that parts of the coolant channel 23 can also be formed by one or more parts which are separate from the housing and arranged inside the housing.

The plasma-generating device 1 can be provided with a coolant channel 23 which is provided with one or more outlet openings 25. Alternatively, the plasma-generating device 1 can be provided with a plurality of coolant channels 23, which each can be provided with one or more outlet openings 25. Each coolant channel 23 can also be divided into a plurality of channel portions which are combined in a common channel portion, which common channel portion can be provided with one or more outlet openings 25. It is also possible to use all or some of the channels 23 for other purposes. For example, three channels 23 can be arranged, two being used to be passed by coolant and one to suck liquids, or the like, from a surgical area etc.

In the embodiment shown in Fig. 1a, a part of the coolant channel 23 extends through the end sleeve 3 and around the intermediate electrodes 9', 9", 9"'. The coolant channel 23 according to Fig. 1a is provided with a plurality of outlet openings 25.

Moreover the outlet openings 25 of the coolant channel 23 are arranged beyond, in the direction from the cathode 5 to the anode 7, the intermediate electrodes 9', 9", 9"'. In the embodiment shown in Fig. 1a, the coolant channel 23 extends through the end sleeve 3 and the anode 7. Moreover the channel direction of the coolant channel 23 at the outlet openings 25 has a directional component which is the same as that of the channel direction of the plasma channel 11 at the opening thereof. According to Fig. 1a, two such outlet openings 25 are shown. Preferably the plasma-generating device 1 is provided with four or more outlet openings 25.

Coolant channels 23 can partly be used to cool the plasma-generating device 1 in operation. As coolant, use is preferably made of water, although other types of fluids are conceivable. To provide cooling, a portion of the coolant channel 23 is arranged so that the coolant is supplied to the end sleeve 3 and flows between the intermediate electrodes 9', 9", 9'" and the inner wall of the end sleeve 3. In operation of the device, it is preferred to let a flow amount of 1-5 ml/s flow through the plasma-generating device 1. The flow amount of coolant may, however, be optionally varied depending on factors such as operating temperature, desired operating properties, field of application etc. In surgical applications, the coolant flow rate is typically between 1 and 3 ml/s and the temperature of the coolant flowing out through the outlet opening 25 is typically between 25 and 40°C.

The coolant which is intended to flow through the coolant channels 25 can also be used to screen the plasma jet and restrict the range of the plasma jet which is emitted through the outlet of the plasma channel 11 in the anode 7. The coolant can also be used to cool areas adjacent to a region, affected by the plasma jet, of an object.

In the embodiment shown in Fig. 1a, the channel direction of the coolant channel 23 at the outlet openings 25 is directed at an angle α towards the centre of the longitudinal direction of the plasma channel 11.

The directed outlet portions allow that the plasma jet generated in operation can be screened in its longitudinal direction by the coolant flowing through the outlet openings 25 of the coolant channel 23. As a result, an operator who operates the device can obtain an essentially distinct position where the plasma jet will be active. In front of this position, suitably little effect from the plasma jet occurs. Consequently this enables good accuracy, for instance, in surgery and other precision-requiring fields of application. At the same time the coolant discharged through the outlet opening 25 of a coolant channel 23 can provide a screening effect in the lateral direction radially outside the centre of the plasma jet. Owing to such screening, a limited surface can be affected by heat locally, and cooled areas of the treated object, outside the area affected by the heat of the plasma, are affected to a relatively small extent by the plasma jet.

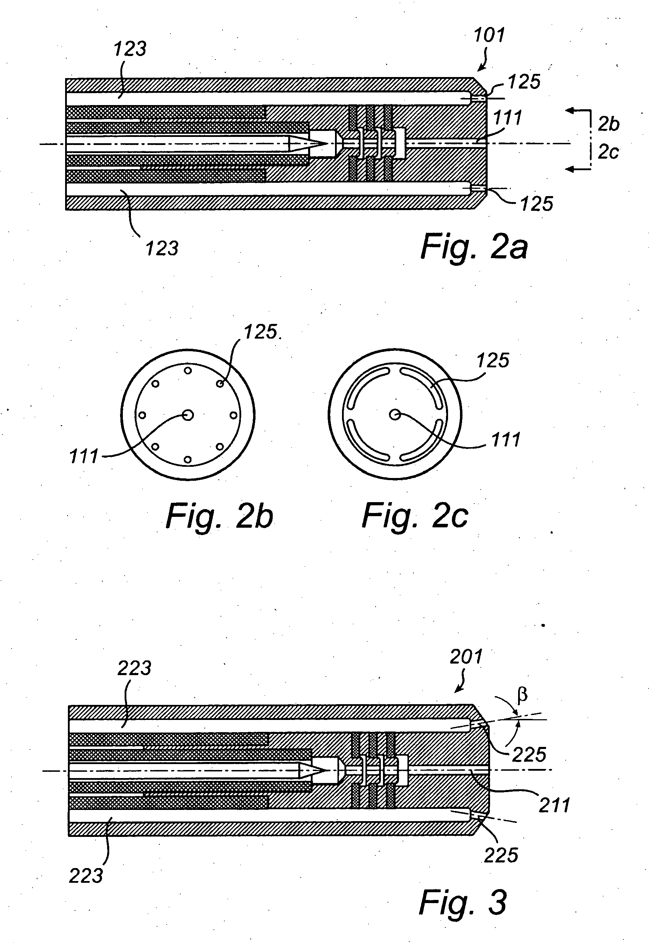

Figs 2a-3 illustrate alternative embodiments of a plasma-generating device 1. Important differences between these embodiments and the embodiment according to Fig. 1a will be described below.

In the embodiment shown in Fig. 2a, the channel direction of the coolant channel 123 at the outlet openings 125 is arranged substantially parallel to the longitudinal direction of the plasma channel 111. In this case, mainly screening of the plasma jet in the radial direction relative to the centre line of the plasma channel 111 is obtained.

Fig. 3 shows another alternative embodiment of a plasma-generating device 201. In the embodiment shown in Fig. 3, the channel direction of the coolant channel 223 at the outlet openings 225 is directed at an angle β away from the centre of the longitudinal direction of the plasma channel 211. This results in screening which increases in distance, relative to the centre line of the plasma channel 211, with an increased distance from the anode 207 and, thus, the outlet of the plasma channel 211.

It will be appreciated that the embodiments according to Figs 1-3 can be combined to form additional embodiments. For example, different outlets can be directed and angled differently in relation to the longitudinal direction of the plasma channel 23; 123; 223. For example, it is possible to provide a plasma-generating device 1; 101; 201 with two outlet portions which are directed parallel to the plasma channel 11; 111; 211 and two outlet portions which are directed inwards to the centre of the longitudinal direction of the plasma channel 11; 111; 211. The variations, with regard to angle and direction of the channel direction of the coolant channel 23; 123; 223 at the outlet openings 25; 125; 225, can be optionally combined depending on the desired properties of the plasma-generating device 1; 101; 201.

It is also possible to vary the angle of the channel direction at the outlet portions 25; 125; 225 in relation to the longitudinal direction of the plasma channel 11; 111; 211. Preferably, the outlet portions are arranged at an angle α, β of ±30 degrees in relation to the longitudinal direction of the plasma channel 11; 111; 211. In the embodiment shown in Fig. 1a the outlet portions are arranged at an angle α of +10 degrees in relation to the longitudinal direction of the plasma channel 11; 111; 211. For the plasma-generating device shown in Fig. 1a, an angle α of 10° means that coolant flowing out through the opening of the coolant channel will intersect the centre of the longitudinal direction of the plasma channel about 8-10 mm in front of the outlet of the plasma channel in the anode.

In the embodiment shown in Fig. 3, the outlet portions are arranged at an angle β of-10 degrees in relation to the longitudinal direction of the plasma channel 11; 111; 211.

Figs 2b-2c are front views of different embodiments of the plasma-generating device 101 in Fig. 2a. Fig. 2b shows a design where the outlet openings 125 of the outlet portions are positioned beside and spaced from the outlet of the plasma channel 111 in the anode. In the embodiment shown in Fig. 2b, the outlet openings 125 are formed as eight circular lead-ins which communicate with the coolant channel 123. It is possible to optionally arrange more or fewer than eight circular lead-ins depending on desirable properties and performance of the plasma-generating device 101. It is also possible to vary the size of the circular lead-ins.

Fig. 2c shows an alternative design of the outlet openings 125 of the coolant channel 123. Fig. 2c is a front view of the plasma-generating device 101 in Fig. 2a. In the embodiment shown in Fig. 2c, the outlet openings 125 are formed as four arched lead-ins which communicate with the coolant channel.

It will be appreciated that the outlet openings 125 of the cooling channel 123 optionally can be designed with a number of alternative geometries and sizes. The cross-sectional surface of the outlet openings can typically be between 0.50 and 2.0 mm², preferably 1 to 1.5 mm².

It is obvious that these different designs of the outlet openings 25; 125; 225 can also be used for the embodiments of the plasma-generating device as shown in Figs 1a-b and 3.

The following description refers to Figs 1a-b. The conditions and dimensions stated are, however, also relevant as exemplary embodiments of the embodiments of the plasma-generating device shown in Figs 2a-3.

The intermediate electrodes 9', 9", 9'" shown in Fig. 1a are arranged inside the end sleeve 3 of the plasma-generating device 1 and are positioned substantially concentrically with the end sleeve 3. The intermediate electrodes 9', 9", 9'" have an outer diameter which in relation to the inner diameter of the end sleeve 3 forms an interspace between the outer surface of the intermediate electrodes 9', 9", 9'" and the inner wall of the end sleeve 3. It is in this space between the intermediate electrodes 9', 9", 9'" and the end sleeve 3 where the coolant flows to be discharged through the outlet openings 125 of the coolant channel 23.

In the embodiment shown in Fig. 1a, three intermediate electrodes 9', 9", 9"', spaced by insulator means 13', 13", 13"', are arranged between the cathode 5 and the anode 7. The first intermediate electrode 9', the first insulating 13' and the second intermediate electrode 9" are suitably press-fitted to each other. Similarly, the second intermediate electrode 9", the second insulator 13" and the third intermediate electrode 9'" are suitably press-fitted to each other. However, it will be appreciated that the number of intermediate electrodes 9', 9", 9'" can be optionally selected depending on the desired purpose.

The intermediate electrode 9'" which is positioned furthest away from the cathode 5 is in contact with an annular insulator means 13'" which is arranged against the anode 7.

The anode 7 is connected to the elongate end sleeve 3. In the embodiment shown in Fig. 1a, the anode 7 and the end sleeve 3 are integrally formed with each other. In alternative embodiments, the anode 7 can be designed as a separate element which is joined to the end sleeve 3 by a threaded joint between the anode and the end sleeve, by welding or by soldering. The connection between the anode 7 and the end sleeve 3 is suitably such as to provide electrical contact between the two.

Suitable geometric relationships between parts included in the plasma-generating device 1, 101, 201 will be described below with reference to Figs 1a-b. It should be noted that the dimensions stated below merely constitute exemplary embodiments of the plasma-generating device 1, 101, 201 and can be varied depending on the field of application and the desired properties. It should also be noted that the examples described in Figs 1a-b can also be applied to the embodiments in Figs 2a-3.

The inner diameter dᵢ of the insulator element 19 is only slightly greater than the outer diameter d_{c} of the cathode 5. In one embodiment, the difference in cross-section, in a common cross-section, between the cathode 5 and the inner diameter dᵢ of the insulator element 19 is suitably equal to or greater than a minimum cross-section of the plasma channel 11. Such a cross-section of the plasma channel 11 can be positioned anywhere along the extent of the plasma channel 11.

In the embodiment shown in Fig. 1b, the outer diameter d_{c} of the cathode 5 is about 0.50 mm and the inner diameter dᵢ of the insulator element about 0.80 mm.

In one embodiment, the cathode 5 is arranged so that a partial length of the cathode tip 15 projects beyond a boundary surface 21 of the insulator element 19. The tip 15 of the cathode 5 is in Fig. 1b positioned so that about half the length L_{c} of the tip 15 projects beyond the boundary surface 21 of the insulator element 19. In the embodiment shown in Fig. 1b, this projection l_{c} corresponds to approximately the diameter d_{c} of the cathode 5.

The total length L_{c} of the cathode tip 15 is suitably greater than 1.5 times the diameter d_{c} of the cathode 5 at the base of the cathode tip 15. Preferably the total length L_{c} of the cathode tip 15 is about 1.5-3 times the diameter d_{c} of the cathode 5 at the base of the cathode tip 15. In the embodiment shown in Fig. 1b, the length L_{c} of the cathode tip 15 corresponds to about 2 times the diameter d_{c} of the cathode 5 at the base of the cathode tip 15.

In one embodiment, the diameter d_{c} of the cathode 5 is about 0.3-0.6 mm at the base of the cathode tip 15. In the embodiment shown in Fig. 1b, the diameter d_{c} of the cathode 5 is about 0.50 mm at the base of the cathode tip 15. Preferably the cathode has a substantially identical diameter d_{c} between the base of the cathode tip 15 and the end of the cathode 5 opposite the cathode tip 15.

However, it will be appreciated that it is possible to vary this diameter d_{c} along the extent of the cathode 5. In one embodiment, the plasma chamber 17 has a diameter D_{c} which corresponds to approximately 2-2.5 times the diameter d_{c} of the cathode 5 at the base of the cathode tip 15. In the embodiment shown in Fig. 1b, the plasma chamber 17 has a diameter D_{ch} which corresponds to approximately 2 times the diameter d_{c} of the cathode 5.

The extent L_{ch} of the plasma chamber 17 in the longitudinal direction of the plasma-generating device 1 corresponds to approximately 2-2.5 times the diameter d_{c} of the cathode 5 at the base of the cathode tip 15. In the embodiment shown in Fig. 1b, the length L_{ch} of the plasma chamber 17 corresponds to approximately the diameter D_{ch} of the plasma chamber 17.

In one embodiment the tip 15 of the cathode 5 extends over half the length L_{ch} of the plasma chamber 17 or more than said length. In an alternative embodiment, the tip 15 of the cathode 5 extends over 1/2 to 2/3 of the length L_{ch} of the plasma chamber 17. In the embodiment shown in Fig. 1b, the cathode tip 15 extends approximately over half the length L_{ch} of the plasma chamber 17.

In the embodiment shown in Fig. 1b, the cathode 5 extending into the plasma chamber 17 is positioned at a distance from the end of the plasma chamber 17 closest to the anode 7 which corresponds to approximately the diameter d_{c} of the cathode 5 at the base thereof.

In the embodiment shown in Fig. 1b, the plasma chamber 17 is in fluid communication with the plasma channel 11. The plasma channel 11 suitably has a diameter d_{ch} which is about 0.2-0.5 mm. In the embodiment shown in Fig. 1b, the diameter d_{ch} of the plasma channel 11 is about 0.40 mm. However, it will be appreciated that the diameter d_{ch} of the plasma channel 11 can be varied in different ways along the extent of the plasma channel 11 to provide different desirable properties.

A transition portion 27 is arranged between the plasma chamber 17 and the plasma channel 11 and constitutes a tapering transition, in the direction from the cathode 5 to the anode 7, between the diameter D_{ch} of the plasma chamber 17 and the diameter d_{ch} of the plasma channel 11. The transition portion 27 can be formed in a number of alternative ways. In the embodiment shown in Fig. 1b, the transition portion 27 is formed as a bevelled edge which forms a transition between the inner diameter D_{ch} of the plasma chamber 17 and the inner diameter d_{ch} of the plasma channel 11. However, it should be noted that the plasma chamber 17 and the plasma channel 11 can be arranged in direct contact with each other without a transition portion 27 arranged between the two. The use of a transition portion 27 as shown in Fig. 1b allows advantageous heat extraction to cool structures adjacent to the plasma chamber 17 and the plasma channel 11.

The plasma channel 11 is formed by the anode 7 and the intermediate electrodes 9', 9", 9"' arranged between the cathode 5 and the anode 7. The length of the plasma channel 11 between the opening of the plasma channel closest to the cathode and up to the anode corresponds suitably to about 4-10 times the diameter d_{ch} of the plasma channel 11. In the embodiment shown in Fig. 1a, the length of the plasma channel 11 between the opening of the plasma channel closest to the cathode and the anode is about 1.6 mm.

That part of the plasma channel which extends through the anode is about 3-4 times the diameter d_{ch} of the plasma channel 11. For the embodiment shown in Fig. 1a, that part of the plasma channel which extends through the anode has a length of about 2 mm.

The plasma-generating device 1 can advantageously be provided as a part of a disposable instrument. For example, a complete device with the plasma-generating device 1, outer shell, tubes, coupling terminals etc. can be sold as a disposable instrument. Alternatively, only the plasma-generating device 1 can be disposable and connected to multiple-use devices.

Other embodiments and variants are conceivable within the scope of the present invention. For example, the number and shape of the electrodes 9', 9", 9'" can be varied according to which type of plasma-generating gas is used and which properties of the generated plasma are desired.

In use the plasma-generating gas, such as argon, which is supplied through the gas supply part, is introduced into the space between the cathode 5 and the insulator element 19 as described above. The supplied plasma-generating gas is passed on through the plasma chamber 17 and the plasma channel 11 to be discharged through the opening of the plasma channel 11 in the anode 7. Having established the gas supply, a voltage system is switched on, which initiates a discharge process in the plasma channel 11 and establishes an electric arc between the cathode 5 and the anode 7. Before establishing the electric arc, it is suitable to supply coolant to the plasma-generating device 1 through the coolant channel 23, as described above. Having established the electric arc, a gas plasma is generated in the plasma chamber 17, which during heating is passed on through the plasma channel 11 to the opening thereof in the anode 7.

A suitable operating current for the plasma-generating devices 1, 101, 201 according to Figs 1-3 is 4-10 ampere, preferably 4-6 ampere. The operating voltage of the plasma-generating device 1, 101, 201 is, inter alia, dependent on the number of intermediate electrodes and the length thereof. A relatively small diameter of the plasma channel allows relatively low consumption of energy and relatively low operating current in use of the plasma-generating device 1, 101, 201.

In the electric arc established between the cathode and anode, there prevails in the centre thereof, along the centre axis of the plasma channel, a temperature T which is proportional to the relationship between the discharge current I and the diameter d_{ch} of the plasma channel (T=k*i/d_{ch}). To provide, at a relatively low current level, a high temperature of the plasma, for instance 10,000 to 15,000°C, at the outlet of the plasma channel in the anode, the cross-section of the plasma channel and, thus, the cross-section of the electric arc which heats the gas should be small, for instance 0.2-0.5 mm. With a small cross-section of the electric arc, the electric field strength in the channel has a high value.

## Claims

1. A plasma surgical device comprising a plasma-generating device (1), said plasma-generating device (1, 101, 201) comprising:
an anode (5);
a cathode (7);
a plasma channel (11, 111, 211) extending longitudinally between said cathode and through said anode, having an outlet opening at the end furthest from the cathode;
at least one intermediate electrode (9', 9", 9"') arranged at least partly between said anode and said cathode, said at least one intermediate electrode and said anode forming at least a part of the plasma channel, said at least one intermediate electrode being electrically insulated from each other and said anode; and
at least one coolant channel (23, 123, 223) extending longitudinally in the device, , whereby a coolant liquid flowing through said coolant channel cools a portion of the device to which the at least one coolant channel is adjacent,
**characterized in that** said at least one coolant channel (23, 123, 223) has at least one outlet opening (25, 125, 225) at the end closest to the anode and
said at least one outlet opening (25, 125, 225) of the coolant channel (23, 123, 223) is arranged in close proximity to the outlet opening of said plasma channel at the end furthest from the cathode, whereby the coolant liquid flowing out of said at least one outlet opening of the coolant channel restricts an area of a plasma effect.

2. The plasma surgical device comprising a plasma-generating device of claim 1, in which the outlet opening (25, 125, 225) of the coolant channel (23, 123, 223) is arranged in the anode (5).

3. The plasma surgical device comprising a plasma-generating device of claim 1, in which a substantial portion of said coolant channel (23, 123, 223) is substantially parallel to said plasma channel (11, 111,211).

4. The plasma surgical device comprising a plasma-generating device of claim 1, in which an angle (α, β) of a direction of the coolant channel (23, 123, 223) at said outlet opening (25, 125, 225) of the coolant channel relative to a direction of said plasma channel (11, 125, 211) at the opening of said plasma channel furthest from the cathode is between +30 and -30 degrees.

5. The plasma surgical device comprising a plasma-generating device (101) of claim 4, in which a direction of the coolant channel (123) at said outlet opening (125) of the coolant channel is substantially parallel to a direction of the plasma channel (111) at the outlet opening of the plasma channel furthest from the cathode.

6. The plasma surgical device comprising a plasma-generating device (1) of claim 4, in which the coolant channel (23) at said outlet opening (25) of the coolant channel angles toward the plasma channel.

7. The plasma surgical device comprising a plasma-generating device (201) of claim 4, in which the coolant channel (223) at said outlet opening (225) of the coolant channel angles away from the plasma channel.

8. The plasma surgical device comprising a plasma-generating device of claim 1, in which during operation the coolant liquid flows through said coolant channel in the direction from the cathode to the anode.

9. The plasma surgical device comprising a plasma-generating device of claim 1, in which a part of said coolant channel (23, 123, 223) extends along said at least one intermediate electrode (9', 9", 9"').

10. The plasma surgical device comprising a plasma-generating device of claim 1, in which a part of said coolant channel (23, 123, 223) extends along the outer periphery of said at least one intermediate electrode (9', 9", 9"').

11. The plasma surgical device comprising a plasma-generating device of claim 1 further comprising a sleeve (3) connected to the anode (7), which forms a part of a radially outwardly positioned boundary surface of the coolant channel (23, 123, 223).

12. The plasma surgical device comprising a plasma-generating device of claim 1, in which said at least one intermediate electrode (9', 9", 9"') forms a part of a radially inwardly positioned boundary surface of the coolant channel (23, 123, 223).

13. The plasma surgical device comprising a plasma-generating device of claim 1, in which during operation the coolant liquid flows through said coolant channel (23, 123, 223) with a rate of between 1 and 5 ml/s.

14. The plasma surgical device comprising a plasma-generating device of claim 1, in which said at least one coolant channel (23, 123, 223) has at least two outlet openings (25, 125, 225).

15. The plasma surgical device comprising a plasma-generating device of claim 14, in which said at least two outlet openings of said at least one outlet opening (25, 125, 225) are arranged around said outlet opening of the plasma channel (11, 111, 211).

16. The plasma surgical device comprising a plasma-generating device of claim 15, in which said at least one coolant channel has at least four outlet openings.

17. The plasma surgical device comprising a plasma-generating device of claim 16, in which a cross-section of the opening of the at least one coolant channel is elongated.

18. The plasma surgical device comprising a plasma-generating device of claim 1 comprising two or more coolant channels (23, 123, 223).

## Patentansprüche

1. Plasmachirurgievorrichtung, welche eine Plasmaerzeugungseinrichtung (1) aufweist, wobei die Plasmaerzeugungseinrichtung (1, 101, 201) Folgendes aufweist:
eine Anode (5);
eine Kathode (7);
einen Plasmakanal (11, 111, 211), der sich längs zwischen der Kathode und durch die Anode erstreckt und eine Auslassöffnung am von der Kathode am weitesten entfernten Ende aufweist;
mindestens eine Zwischenelektrode (9', 9", 9"'), die mindestens teilweise zwischen der Anode und der Kathode angeordnet ist, wobei die mindestens eine Zwischenelektrode und die Anode mindestens einen Teil des Plasmakanals bilden, wobei die mindestens eine Zwischenelektrode voneinander und von der Anode elektrisch isoliert ist; und
mindestens einen Kühlmittelkanal (23, 123, 223), der sich längs in der Vorrichtung erstreckt, wodurch eine durch den Kühlmittelkanal fließende Kühlmittelflüssigkeit einen Teil der Vorrichtung abkühlt, an den der mindestens eine Kühlmittelkanal angrenzt,
**dadurch gekennzeichnet, dass** der mindestens eine Kühlmittelkanal (23, 123, 223) mindestens eine Auslassöffnung (25, 125, 225) am zur Anode dichtesten Ende aufweist und
die mindestens eine Auslassöffnung (25, 125, 225) des Kühlmittelkanals (23, 123, 223) in enger Nachbarschaft zur Auslassöffnung des Plasmakanals am von der Kathode am weitesten entfernten Ende angeordnet ist, wodurch die aus der mindestens einen Auslassöffnung des Kühlmittelkanals ausfließende Kühlmittelflüssigkeit einen Bereich eines Plasmaeffekts begrenzt.

2. Plasmachirurgievorrichtung, welche eine Plasmaerzeugungseinrichtung nach Anspruch 1 aufweist, wobei die Auslassöffnung (25, 125, 225) des Kühlmittelkanals (23, 123, 223) in der Anode (5) angeordnet ist.

3. Plasmachirurgievorrichtung, welche eine Plasmaerzeugungseinrichtung nach Anspruch 1 aufweist, wobei ein wesentlicher Teil des Kühlmittelkanals (23, 123, 223) im Wesentlichen parallel zum Plasmakanal (11, 111, 211) verläuft.

4. Plasmachirurgievorrichtung, welche eine Plasmaerzeugungseinrichtung nach Anspruch 1 aufweist, wobei ein Winkel (α, β) einer Richtung des Kühlmittelkanals (23, 123, 223) an der Auslassöffnung (25, 125, 225) des Kühlmittelkanals relativ zu einer Richtung des Plasmakanals (11, 111, 211) an der von der Kathode am weitesten entfernten Öffnung des Plasmakanals zwischen +30 und -30 Grad beträgt.

5. Plasmachirurgievorrichtung, welche eine Plasmaerzeugungseinrichtung (101) nach Anspruch 4 aufweist, wobei eine Richtung des Kühlmittelkanals (123) an der Auslassöffnung (125) des Kühlmittelkanals im Wesentlichen parallel zu einer Richtung des Plasmakanals (111) an der von der Kathode am weitesten entfernten Auslassöffnung des Plasmakanals verläuft.

6. Plasmachirurgievorrichtung, welche eine Plasmaerzeugungseinrichtung (1) nach Anspruch 4 aufweist, wobei der Kühlmittelkanal (23) an der Auslassöffnung (25) des Kühlmittelkanals zum Plasmakanal hin abgewinkelt ist.

7. Plasmachirurgievorrichtung, welche eine Plasmaerzeugungseinrichtung (201) nach Anspruch 4 aufweist, wobei der Kühlmittelkanal (223) an der Auslassöffnung (225) des Kühlmittelkanals vom Plasmakanal weg abgewinkelt ist.

8. Plasmachirurgievorrichtung, welche eine Plasmaerzeugungseinrichtung nach Anspruch 1 aufweist, wobei im Betrieb die Kühlmittelflüssigkeit durch den Kühlmittelkanal in der Richtung von der Kathode zur Anode fließt.

9. Plasmachirurgievorrichtung, welche eine Plasmaerzeugungseinrichtung nach Anspruch 1 aufweist, wobei ein Teil des Kühlmittelkanals (23, 123, 223) sich entlang der mindestens einen Zwischenelektrode (9', 9", 9"') erstreckt.

10. Plasmachirurgievorrichtung, welche eine Plasmaerzeugungseinrichtung nach Anspruch 1 aufweist, wobei ein Teil des Kühlmittelkanals (23, 123, 223) sich entlang dem äußeren Umfang der mindestens einen Zwischenelektrode (9', 9", 9"') erstreckt.

11. Plasmachirurgievorrichtung, welche eine Plasmaerzeugungseinrichtung nach Anspruch 1 und ferner eine mit der Anode (7) verbundene Hülse (3) aufweist, die einen Teil einer radial nach außen positionierten Grenzfläche des Kühlmittelkanals (23, 123, 223) bildet.

12. Plasmachirurgievorrichtung, welche eine Plasmaerzeugungseinrichtung nach Anspruch 1 aufweist, wobei die mindestens eine Zwischenelektrode (9', 9", 9"') einen Teil einer radial nach innen positionierten Grenzfläche des Kühlmittelkanals (23, 123, 223) bildet.

13. Plasmachirurgievorrichtung, welche eine Plasmaerzeugungseinrichtung nach Anspruch 1 aufweist, wobei im Betrieb die Kühlmittelflüssigkeit durch den Kühlmittelkanal (23, 123, 223) mit einem Durchfluss zwischen 1 und 5 ml/s fließt.

14. Plasmachirurgievorrichtung, welche eine Plasmaerzeugungseinrichtung nach Anspruch 1 aufweist, wobei der mindestens eine Kühlmittelkanal (23, 123, 223) mindestens zwei Auslassöffnungen (25, 125, 225) aufweist.

15. Plasmachirurgievorrichtung, welche eine Plasmaerzeugungseinrichtung nach Anspruch 14 aufweist, wobei die mindestens zwei Auslassöffnungen (25, 125, 225) der mindestens einen Auslassöffnung (25, 125, 225) um die Auslassöffnung des Plasmakanals (11, 111, 211) herum angeordnet sind.

16. Plasmachirurgievorrichtung, welche eine Plasmaerzeugungseinrichtung nach Anspruch 15 aufweist, wobei der mindestens eine Kühlmittelkanal mindestens vier Auslassöffnungen aufweist.

17. Plasmachirurgievorrichtung, welche eine Plasmaerzeugungseinrichtung nach Anspruch 16 aufweist, wobei ein Querschnitt der Öffnung des mindestens einen Kühlmittelkanals länglich ausgebildet ist.

18. Plasmachirurgievorrichtung, welche eine Plasmaerzeugungseinrichtung nach Anspruch 1 aufweist, welche zwei oder mehr Kühlmittelkanäle (23, 123, 223) aufweist.

## Revendications

1. Dispositif chirurgical à plasma comprenant un dispositif de génération de plasma (1), ledit dispositif de génération de plasma (1, 101, 201) comprenant :
une anode (5) ;
une cathode (7),
un canal à plasma (11, 111, 211) s'étendant longitudinalement entre ladite cathode et à travers ladite anode, ayant une ouverture de sortie au niveau de l'extrémité la plus éloignée de ladite cathode ;
au moins une électrode intermédiaire (9', 9", 9"') agencée au moins partiellement entre ladite anode et ladite cathode, ladite au moins une électrode intermédiaire et ladite anode formant au moins une partie du canal à plasma, ladite au moins une électrode intermédiaire étant électriquement isolée l'une de l'autre et de ladite anode; et
au moins un canal de refroidissement (23, 123, 223) s'étendant longitudinalement dans le dispositif, **caractérisé en ce que** ledit au moins canal de refroidissement (23, 123, 223) a au moins une ouverture de sortie (25, 125, 225) au niveau de l'extrémité la plus proche de l'anode, selon lequel un liquide de refroidissement circulant à travers ledit canal de refroidissement refroidit une partie du dispositif auquel l'au moins un canal de refroidissement est adjacent, et
ladite au moins une ouverture de sortie (25, 125, 225) du canal de refroidissement (23, 123, 223) est agencée à proximité étroite avec l'ouverture de sortie dudit canal à plasma au niveau de l'extrémité la plus éloignée de la cathode, selon lequel le liquide de refroidissement s'écoulant hors de ladite au moins une ouverture de sortie du canal de refroidissement restreint une surface d'un effet plasma.

2. Dispositif chirurgical à plasma comprenant un dispositif de génération de plasma selon la revendication 1, dans lequel l'ouverture de sortie (25, 125, 225) du canal de refroidissement (23, 123, 223) est agencée dans l'anode (5).

3. Dispositif chirurgical à plasma comprenant un dispositif de génération de plasma selon la revendication 1, dans lequel une partie importante dudit canal de refroidissement (23, 123, 223) est sensiblement parallèle audit canal à plasma (11, 111, 211).

4. Dispositif chirurgical à plasma comprenant un dispositif de génération de plasma selon la revendication 1, dans lequel un angle (α, β) d'une direction du canal de refroidissement (23, 123, 223) au niveau de ladite ouverture de sortie (25, 125, 225) du canal de refroidissement par rapport à une direction dudit canal à plasma (11, 125, 211) au niveau de l'ouverture dudit canal à plasma la plus éloigné de la cathode est compris entre +30 et -30 degrés.

5. Dispositif chirurgical à plasma comprenant un dispositif de génération de plasma (101) selon la revendication 4, dans lequel une direction du canal de refroidissement (123) au niveau de l'ouverture de sortie (125) du canal de refroidissement est sensiblement parallèle à une direction du canal à plasma (111) au niveau de l'ouverture de sortie du canal à plasma la plus éloignée de la cathode.

6. Dispositif chirurgical à plasma comprenant un dispositif de génération de plasma (1) selon la revendication 4, dans lequel le canal de refroidissement (23) au niveau de l'ouverture de sortie (25) du canal de refroidissement est dans l'angle du canal à plasma.

7. Dispositif chirurgical à plasma comprenant un dispositif de génération de plasma (201) selon la revendication 4, dans lequel le canal de refroidissement (223) au niveau de l'ouverture de sortie (225) du canal de refroidissement n'est pas dans l'angle du canal à plasma.

8. Dispositif chirurgical à plasma comprenant un dispositif de génération de plasma selon la revendication 1, dans lequel, pendant le fonctionnement, le liquide de refroidissement circule à travers ledit canal de refroidissement dans la direction de la cathode à l'anode.

9. Dispositif chirurgical à plasma comprenant un dispositif de génération de plasma selon la revendication 1, dans lequel une partie dudit canal de refroidissement (23, 123, 223) s'étend le long de ladite au moins une électrode intermédiaire (9', 9", 9"').

10. Dispositif chirurgical à plasma comprenant un dispositif de génération de plasma selon la revendication 1, dans lequel une partie dudit canal de refroidissement (23, 123, 223) s'étend le long de la périphérie extérieure de ladite au moins une électrode intermédiaire (9', 9", 9"').

11. Dispositif chirurgical à plasma comprenant un dispositif de génération de plasma selon la revendication 1, comprenant en outre un manchon (3) raccordé à l'anode (7), qui forme une partie d'une surface limite positionnée radialement vers l'extérieur du canal de refroidissement (23, 123, 223).

12. Dispositif chirurgical à plasma comprenant un dispositif de génération de plasma selon la revendication 1, dans lequel ladite au moins une électrode intermédiaire (9', 9", 9"') forme une partie d'une surface limite positionnée radialement vers l'intérieur du canal de refroidissement (23, 123, 223).

13. Dispositif chirurgical à plasma comprenant un dispositif de génération de plasma selon la revendication 1, dans lequel, pendant le fonctionnement, le liquide de refroidissement circule à travers ledit canal de refroidissement (23, 123, 223) avec un débit compris entre 1 et 5 ml/s.

14. Dispositif chirurgical à plasma comprenant un dispositif de génération de plasma selon la revendication 1, dans lequel ledit au moins un canal de refroidissement (23, 123, 223) a au moins deux ouvertures de sortie (25, 125, 225).

15. Dispositif chirurgical à plasma comprenant un dispositif de génération de plasma selon la revendication 14, dans lequel lesdites au moins deux ouvertures de sortie de ladite au moins une ouverture de sortie (25, 125, 225) sont agencées autour de ladite ouverture de sortie du canal à plasma (11, 111, 211).

16. Dispositif chirurgical à plasma comprenant un dispositif de génération de plasma selon la revendication 15, dans lequel ledit au moins un canal de refroidissement a au moins quatre ouvertures de sortie.

17. Dispositif chirurgical à plasma comprenant un dispositif de génération de plasma selon la revendication 16, dans lequel une section transversale de l'ouverture de l'au moins un canal de refroidissement est allongée.

18. Dispositif chirurgical à plasma comprenant un dispositif de génération de plasma selon la revendication 1, comprenant au moins deux canaux de refroidissement (23, 123, 223).
